# EUROPÄISCHE PATENTSCHRIFT

(11) **EP 0 406 623 B1**
(45) Veröffentlichungstag und Bekanntmachung des Hinweises auf die Patenterteilung: **30.11.1994**
(21) Anmeldenummer: 90111715.0
(22) Anmeldetag: 21.06.1990
(51) Int. Cl.: A61K 9/28, A61K 9/20, A61K 9/48, A61K 9/52, A23P 1/08, A23G 3/30, A23G 3/00, A23L 1/317, A61K 9/50

(54) **Polyasparaginsäurederivate als Überzugsmittel für Arzneiformen und Lebensmittel**
Polyaspartic acid derivatives as a coating for pharmaceutical forms and foodstuffs
Dérivés de l'acide polyaspartique comme agents d'enrobage pour des formes pharmaceutiques et des aliments

(30) Priorität: 04.07.1989 DE 3921912
(43) Veröffentlichungstag der Anmeldung: 09.01.1991
(73) Patentinhaber: Röhm GmbH, 64293 Darmstadt (DE)
(72) Erfinder: Lehmann, Klaus, Dr., D-6101 Rossdorf 1 (DE); Jelitte, Rüdiger, Dr., D-6101 Rossdorf 1 (DE); Knebel, Joachim, Dr., D-6100 Darmstadt (DE)

(56) Entgegenhaltungen:
- EP-A- 0 274 127
- EP-A- 0 439 846
- DE-A- 3 612 102
- FR-A- 2 520 229

## Beschreibung

### Gebiet der Erfindung

Die Erfindung betrifft biologisch verträgliche Polyasparaginsäurederivate und ihre Verwendung vor allem bei pharmazeutischen und lebensmitteltechnischen Zubereitungen.

### Stand der Technik

Sowohl in der pharmazeutischen Technologie als auch in der Lebensmitteltechnologie werden polymere Substanzen als Zusatzstoffe in großem Umfang verwendet. Diese Stoffe sind bei diesen Anwendungen selbst keine Arzneimittel bzw. Lebensmittel, sind aber für spezifische Anwendungen der Wirksubstanzen oder für z.B. spezielle Verarbeitungen von Lebensmittel unentbehrlich.

Polymere, filmbildende Substanzen werden beispielsweise zur Verbesserung der Handhabbarkeit von pharmazeutischen Zubereitungen, zur Verbesserung ihrer Lagerstabilität und vor allem zur Beeinflussung der Abgabegeschwindigkeit der Wirksubstanzen bei deren medikamentösen Anwendung eingesetzt. Verwendet werden synthetische Polymere und chemisch modifizierte Naturstoffe wie Polyethylenglykol, Polyvinylpyrrolidon, Polymethacrylate, Polyvinylacetat und Celluloseester und -ether.

Für spezielle Anwendungen in der Medizin und Pharmazie gibt es schon eine Vielzahl synthetisch hergestellter Polypeptide. Abgesehen von Peptiden mit wirkungsspezifischer Sequenz, sind dies auch Polymere aus einzelnen Aminosäuren, wie vor allem Polyasparaginsäure, Polyglutaminsäure und Polylysin, sowie Mischpolymerisate der diesen Polyaminosäuren zugrunde liegenden Monomerbausteinen. Diese Polymeren besitzen keine besonderen biologischen Wirksamkeiten und wurden als Plasmaexpander vorgeschlagen.
Auch Derivate solcher Polyaminosäuren sind bekannt und biologisch gut verträglich. So ist das α,β-Poly-(2-hydroxyethyl)-DL-Aspartamid als Plasmaexpander einsetzbar (P.Neri et al. Journal of Medicinal Chemistry, 1973, Vol. 16, S. 893 - 897). In der DE-OS 37 00 128 sind Acylderivate des α,β-Poly-(2-hydroxyethyl)-DL-Aspartamids und allgemein von α,β-Poly-(hydroxyalkyl)-DL-Aspartamiden beschrieben. Durch Einbau geeigneter biologisch inaktiver Acylgruppen läßt sich die Abbaugeschwindigkeit dieser Polymeren in vivo in gewünschter Weise steuern. Sie eignen sich so zum Einsatz abbaubarer Arzneistoffimplantate mit kontrollierter Wirkstoffabgabe. Die Wirkstoffe sind in die Polymermatrix eingebettet.
Lösliche und biologisch abbaubare Mischpolymerisate aus Asparaginsäure- und/oder Glutaminsäure-Einheiten, die reaktive Gruppen, wie z.B. Hydrazid- oder Azidgruppen, für die chemische Anbindung von biologisch aktiven Stoffen enthalten, sind aus der DE-OS 36 12 102 bekannt.

Aus FR-A 2 520 229 sind Arzneimittelüberzüge und -matrixbinder auf Basis von Polypeptiden bekannt; beispielsweise wird Polyasparaginsäure eingesetzt.

Für eine gezielte Freisetzung von Wirkstoffen an oder in die Nähe ihrer Wirkorte werden Arzneimittel vielfach in Form überzogener Tabletten appliziert. Alle bisher bekannten Polyaminosäuren und deren Derivate sind als Überzugsmittel für Arzneiformen und auch für Lebensmittel trotz vorhersehbarer Vorteile, noch nicht eingesetzt worden. Dies kann kostenbegründet sein oder in den Eigenschaften der bisher bekannten Polyaminosäuren und deren Derivate liegen. So ist das aus dem Stand der Technik her bekannte α,β-Poly-(2-hydroxyethyl)-DL-Aspartamid für den Anwendungsbereich Überzüge für Arzneiformen und Lebensmittel zu hydrophil.

### Aufgabe und Losung

Es stellte sich nun die Aufgabe, biologisch verträgliche,aus natürlichen Bausteinen aufgebaute Polymere zu finden, die filmbildend sind und sich für die Herstellung von Überzugsmittel im pharmazeutischen und Lebensmittelbereich einsetzen lassen, und die kostengünstig aus gut zugänglichen, niedermolekularen Substanzen herstellbar sind.

Es wurde gefunden, daß synthetische Polymere mit Einheiten von Asparaginsäurederivaten, die durch Umsetzung von Polysuccinimid mit Verbindungen der Formel 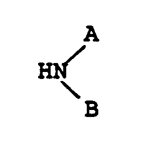
in der A und B Wasserstoff und/oder organische Reste aus Alkyl- bzw. Alkylengruppen sind, die noch weitere funktionelle Gruppen enthalten können, unter Ringöffnung erhalten werden, für das Überziehen von Lebensmittel und Arzneiformen geeignet sind, und daß ihre spezifische Löslichkeit unter den Anwendungsbedingungen durch die funktionellen Gruppen der erfindungsgemäßen Retardierungsmittel eingestellt werden kann. Polypeptide, aufgebaut aus natürlich vorkommenden Aminosäuren, haben als filmbildende Substanzen Vorteile, die in ihrer guten biologischen Verträglichkeit und der physiologischen Unbedenklichkeit der zu ihrer Herstellung verwendeten Ausgangsstoffe liegen, so daß Verbraucher, vor allem gesundheitlich beeinträchtigte Patienten davon unbelastet bleiben. Über die galenischen Eigenschaften von Tabletten, insbesondere ihren Zerfall in Wasser bzw. in den Verdauungssäften des Magens oder des Darms, und damit über die charakteristische Freisetzungsrate, entscheiden im wesentlichen die chemischen und physikalischen Eigenschaften der Überzugssubstanzen.

Die Erfindung betrifft somit die:
Verwendung von Umsetzungsprodukten von Polysuccinimid, die filmbildend sind und Struktureinheiten entsprechend der Formel I 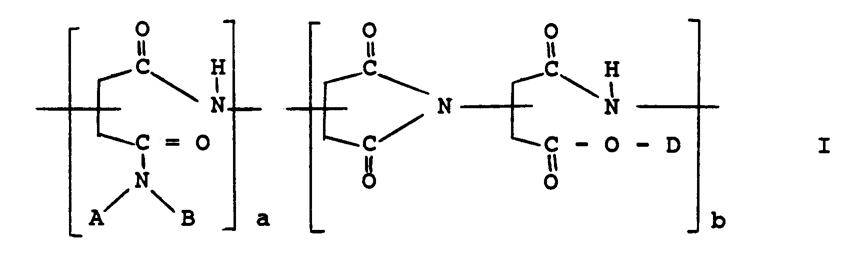
enthalten, in der
- A =: H oder ein Alkylrest bzw. ein Alkylenrest mit 1 bis 8 C-Atomen ist, der auch verzweigt und noch mit cycloaliphatischen oder aromatischen Resten, wobei der ringförmige Substituent auch Heteroatome enthalten kann, oder mit R-O-Gruppen, wobei R = H oder gegebenenfalls eine verzweigte Alkylgruppe oder Cycloalkylgruppe mit 1 bis 10 C-Atomen sein kann, substituiert sein kann,
- B =: H oder ein wie unter A definierter Alkyl- bzw. Alkylenrest ist, der gleich oder verschieden von A sein kann,
- D =: H oder NH₄ oder 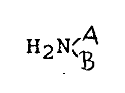 mit den oben angegebenen Bedeutungen von A und B oder ein Alkali und
- a =: 0,2 bis 1 und
- b =: 0,8 bis 0
sind, als Überzugsmittel und/oder Retardierungmittel für Arzneiformen von therapeutischen Wirkstoffen und für Lebensmittel und Genußmittel.
Vorzugsweise weisen die erfindungsgemäß zu verwendenden Polyasparaginsäurederivate a-Werte von 0,5 bis 1 und b-Werte von 0,5 bis 0 auf.

Die Polymeren haben Molekulargewichte von 10 000 bis 200 000, vor allem solche von 30 000 bis 150 000. Sie sind vorzugsweise für die Herstellung von retardierend und gezielt wirkenden Überzügen von Arzneiformen und als Überzugsmittel von Lebensmitteln und Genußmitteln geeignet.

Mit den erfindungsgemäß zu verwendenden Polymeren lassen sich Medikamente und Lebensmittel überziehen, wodurch diese gegenüber äußeren Einflüssen bei der Herstellung, Lagerung und Handhabung durch den Verbraucher zunächst geschützt sind, bei der Einnahme dann aber im Verdauungstrakt gezielt freigesetzt werden.

Je nach dem Charakter der funktionellen Gruppen und den daraus resultierenden Löslichkeitseigenschaften bewirken die Polymerfilme dann eine verlangsamte Auflösung, eine verzögerte Abgabe durch Diffusion oder eine pH-abhängige Freisetzung des Wirkstoffes. Solange die Filme im vorliegenden Milieu des Verdauungstraktes unlöslich sind, schützen sie auch den umhüllten Wirkstoff vor unerwünschten Reaktionen. So können z.B. bitter schmeckende Arzneistoffe im neutralen Milieu des Speichels zurückgehalten und im sauren Magensaft schnell freigegeben, säureempfindliche oder magenreizende Stoffe durch magensaftresistente Filme umhüllt und erst im Darmsaft oberhalb pH 6 freigesetzt und andere spezifische Wirkstoffe sogar erst im Colon oberhalb pH 7 gezielt abgegeben werden. So können Nebenwirkungen vermieden und die therapeutischen Wirkungen verbessert werden. Die Überzugspolymeren lösen sich in dem vorgesehenen Milieu auf und werden auf den natürlichen Stoffwechselwegen ausgeschieden. Durch die Verwendung naturnaher Polymerer als Medikamentenüberzug wird eine Belastung mit körperfremden Materialien und die Gefahr von allergischen und toxischen Reaktionen bei Patienten vermindert.

Bei der Verwendung im Lebensmittelbereich, z.B. für die Umhüllung von Lebensmitteln und Genußmitteln können sie als Materialien ohne Glucosegehalt vorteilhaft, wie beispielsweise zur Kariesvorbeugung, eingesetzt werden.

### Durchführung der Erfindung

Das für die Herstellung der erfindungsgemäß zu verwendenden Polyasparaginsäurederivate einzusetzende Polysuccinimid wird nach bekannten Verfahren, z.B. nach dem von P.Neri et al. in J.Med. Chem. 16, 893 (1973) beschriebenen Verfahren, durch Polykondensation von Asparaginsäure in Gegenwart von Phosphorsäure, synthetisiert. So erhältliches Polysuccinimid, das auch als Polyanhydroasparaginsäure zu bezeichnen ist, ist mit Molekulargewichten von etwa 100 000 (bestimmt durch Viskosimetrie, s. Neri et al.) relativ hochmolekular. Nach den bekannten und bei Neri aufgeführten Herstellungsmethoden, lassen sich Polysuccinimide mit Molekulargewichten im Bereich von 10 000 bis 200 000 erhalten, die sich zur Umsetzung mit Aminen 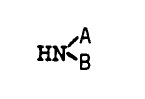
zu filmbildenden und erfindungsgemäß zu verwendenden Polyasparaginsäurederivaten eignen.

Erfindungsgemäß zu verwendende Polyasparaginsäurederivate werden erhalten aus Polysuccinimid und einem oder auch mehreren, d.h. verschiedenen Aminen der allgemeinen Formel 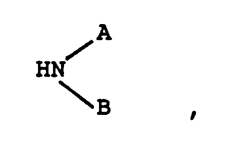
wobei
- A =: H oder ein Alkylrest bzw. ein Alkylenrest mit 1 bis 8 C-Atomen ist, der auch verzweigt und noch mit cycloaliphatischen oder aromatischen Resten, wobei der ringförmige Substituent auch Heteroatome enthalten kann, oder R-O- oder 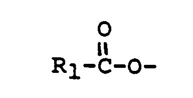 oder 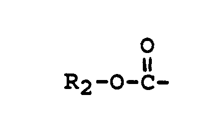 oder R₃R₄N-Gruppen, wobei R, R₁, R₂, R₃ und R₄, H oder gegebenenfalls verzweigte Alkylgruppen oder Cycloalkylgruppen, auch solche mit Sauerstoff- und Stickstoffatomen in der Kette bzw. im Ring, mit 1 bis 10 C-Atomen sein können, substituiert sein kann und
- B =: H oder ein wie unter A definierter Alkyl- bzw. Alkylenrest ist, der gleich oder verschieden von A sein kann,
sind.

Beispielhaft seien Ammoniak und folgende Amine, die der Definition von 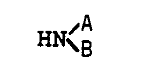
genügen, genannt:
Methylamin, Butylamin, Hexylamin, Ethanolamin, Neopentanolamin, 3-Isononyloxypropylamin, 3-Propanolamin, 2-Methoxyethylamin, 3-Methoxypropoylamin, 3-Ethoxypropylamin, Tetrahydrofurfurylamin, Furfurylamin, Benzylamin, 2-Methoxybenzylamin, Tyramin, Ethylhexoxypropylamin, Monoisopropanolamin, Diethanolamin, Diisopropanolamin, 5-Amino-1-pentanol, 4-Aminocyclohexanol, 2-Aminophenylethanol-1, 1-(2-Aminoethyl)piperazin, N,N-Dimethyl-1,3-propandiamin, 2-Aminopyridin, 3-Aminopyridin, 4-Aminopypridin, N-(3-Aminopropyl)pyrrolidin, Morpholinoethylamin, Glycinethylester (aus diesen oder ähnlichen Estern durch Hydrolyse die Carbonsäurederivate) und andere Aminosäuren und deren Derivate.

Die Umsetzung von Polysuccinimid mit Aminen kann vorteilhaft den Angaben bei Neri entsprechend (dazu auch P. Neri, G. Antoni, Macromol. Synth. 8, 25) schonend bei relativ niedrigen Temperaturen, d.h. bei Temperaturen unter 60 Grad C praktisch bei ca. 20 Grad C durchgeführt werden, um einen Abbau der Polymerkette zu vermeiden. Es hat sich als vorteilhaft für die Einstellung von Eigenschaften der als Überzüge zu verwendenden Polymeren herausgestellt, die polymeranaloge Umsetzung im Bereich von 20 bis 100 Mol%, besonders im Bereich von 30 bis 100 Mol% und vor allem im Bereich von 50 bis 95 Mol% der Succinimidstruktureinheiten durchzuführen. Dazu werden pro Mol Polysuccinimid 0,2 bis 1 Mol eines oder mehrerer Amine 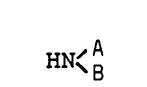
umgesetzt, wobei Polymere mit 20 bis 100 Mol% α,β-D,L-Aspartamid-Einheiten und 80 bis 0 Mol% D,L-Succinimid-Einheiten erhalten werden. Zur Erreichung eines praktisch 100 %igen Derivatisierungsgrades wird die Umsetzung vorteilhaft in Gegenwart von etwa 10 bis 200 Mol% Aminüberschuß durchgeführt.

Die Umsetzung wird normalerweise in Gegenwart eines inerten, polaren aprotischen Lösungsmittels, beispielsweise in Dimethylacetamid, in Dimethylsulfoxid oder vorzugsweise in Dimethylformamid durchgeführt. Gegebenenfalls, wie z.B. bei der Umsetzung von Ethanolamin oder Isopropanolamin, kann die Umsetzung mit dem Polysuccinimid auch ohne zusätzliches Lösemittel ausgeführt werden.
Ist Wasser während oder anschließend an die Umsetzung des Polysuccinimids mit dem Amin 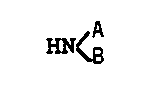
zugegen, so kann das erfindungsgemäß zu verwendende Polymere auch Asparaginsäurebausteine enthalten, die wie in Formel I angegeben, in protonierter Form oder in Salzform vorliegen, bzw. in diese gebracht werden können.

Die Reinigung der polymeren Asparaginsäurederivate, insbesondere die Abtrennung von sonst schwer entfernbaren Lösemitteln, und unumgesetztem Amin, kann durch Ausfällen derselben aus Methanol-Lösungen beim Eintragen in Aceton oder Isopropanol durchgeführt werden.

Für die vorgesehene Anwendung als Überzüge, vor allem als retadierend wirkende Überzüge, lassen sich die Löslichkeitseigenschaften der erfindungsgemäß zu verwendenden Polyasparaginsäurederivate vor allem durch die Struktur der 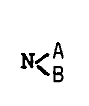
-Seitengruppen beeinflussen. So zeigen z.B. Derivate, die mit Isopropanolamin oder mit Neopentanolamin hergestellt werden, gute Löslichkeiten in Alkoholen oder Wasser, oder solche, die mit Glycinester oder Tetrahydrofurfurylamin erhalten werden, nach Zusatz von 10 bis 20 Gew.-% Wasser, ebenfalls gute Alkohollöslichkeiten. Derivate, die beispielsweise Aminogruppen in der Seitenkette tragen, sind in verdünnten Säuren und damit auch in Magensaft gut löslich. Mit erfindungsgemäß hergestellten Überzügen läßt sich also die Wirkstoffabgabe aus damit hergestellten Produkten gezielt beeinflussen.

Für das Überziehen von Arzneiformen oder Lebensmitteln bzw. Genußmitteln oder für die gesonderte Herstellung von Umhüllungs- bzw. Verpackungsformen, wie z.B. Kapseln oder Folien für Produkte aus dem Arznei-, Lebensmittel- oder Genußmittelbereich, sind die Polyasparaginsäurederivate der Formel I sehr geeignet.

Die neuen Retadierungsmittel für Arzneiformen lassen sich nach bekannten Methoden der pharmazeutischen Technologie (Ullmanns Encyklopädie der technischen Chemie, 4. Auflage, Band 18, Seiten 151 bis 161) aus einem Lösemittel oder aus einer Dispersion unter Filmbildung auf die Substratmaterialien aufbringen. Substratmaterialien sind alle festen Arzneiformen wie Tabletten, Dragees, Pillen, Granulate, Pellets, Kristalle, Pulver oder Kapseln. Aus Polyasparaginsäurederivaten als solche hergestellte Kapseln stehen zur anschließenden Füllung mit z.B pharmakologischen Stoffen zur Verfügung. Für örtliche und transdermale Behandlungen auf der Haut lassen sich Filme und Folien aus Polyasparaginsäureamidderivaten mit Wirkstoffen, wie z.B. Dithranol, z.B. durch Bandgießen herstellen.

Lebensmittel und Genußmittel, die sich erfindungsgemäß überziehen lassen, sind beispielsweise Wurst- und Fleischwaren, Käse, Süßwaren, Zuckerwaren, Kaugummi. Kaugummiüberzüge können so weitgehend zuckerfrei erhalten werden und geben gute Automatenfestigkeit.

Als Überzugsverfahren lassen sich die dazu gebräuchlichen Verfahren, wie das Kesseldragierverfahren oder das Wirbelschicht-Dragierverfahren anwenden, wie sie vor allem in der pharmazeutischen Technologie üblich sind, oder es können die neuen Überzugsmittel zum gleichzeitigen Überziehen und Granulieren von Pulvern verwendet werden, wobei man Granulate enthält, die sich zu Matrixtabletten verpressen oder in Kapseln, auch solchen aus Polyasparaginsäureamidderivaten, füllen lassen.

Auch durch Tauchen und Übergießen von Substraten in bzw. mit Lösungen, Dispersionen oder auch Schmelzen von Polymeren der Formel I oder durch Einsiegeln in nach bekannten Techniken (Ullmann, 4. Auflage, Band 11, Seiten 673 bis 677) getrennt hergestellte Folien aus Polyasparaginsäureamiden mit Dicken von etwa 50 bis 1 000 µm, lassen sich Substrate, vor allem solche größerer Dimensionen, wie sie mehr im Lebensmittelbereich üblich sind, erfindungsgemäß überziehen.

Bei Arzneiformen wird das neue Überzugsmittel im allgemeinen in einer Stärke von 5 bis 50 µm Dicke, vor allem in Schichtdicken von 10 bis 30 µm verwendet. Für die Beschichtung von Granulaten und kleineren Partikeln entspricht dies einem Lackauftrag von etwa 10 bis 20 Gew.-%, bei Tabletten, Dragees oder Kapseln einem Lackauftrag von etwa 3 bis 5 Gew.-%, jeweils bezogen auf das Gewicht des überzogenen Kerns.
Als Zusätze bei der Formulierung des erfindungsgemäßen Überzugsstoffes für anzuwendende Auftragsverfahren, kommen erforderlichenfalls Zusatzstoffe wie z.B. Konservierungsmittel,Farbstoffe und Pigmente, Weichmacher oder Trennmittel in Betracht.

Zur Beschichtung werden die Polymeren der Formel I als etwa 5 bis 25 %ige, besonders als 10 bis 20 %ige Lösungen, in Lösemitteln wie z.B. Methanol, Isopropanol, Aceton, Wasser, Wasser-Alkohol-Gemischen, gegebenenfalls in Gegenwart von gelösten oder suspendierten Zusatzstoffen angewendet. Auch in Form filmbildender Dispersionen, vorteilhaft vor allem als wäßrige Dispersionen, werden die Polymeren zur Herstellung der Überzüge verwendet. Die Dispersionen können durch Zusatz von Fällungsmitteln zu Lösungen der Polyasparaginsäurederivate, wie Wasser zu Lösungen in organischen Lösemitteln, oder wie organische Fällungsmittel zur wäßrigen Lösungen, erhalten werden. Auch durch Dispergieren von Polymerpulvern, wie sie beispielsweise beim Eindampfen von Polymerlösungen oder durch Ausfällungen aus Lösungen erhalten werden, lassen Dispersionen für die erfindungsgemäße Verwendung der Polymeren I zur Herstellung retardierend wirkender Überzüge gewinnen.

Die Erfindung wird durch die folgenden Beispiele, in denen Herstellung und Eigenschaften der erfindungsgemäß zu verwendenden Polymeren und das Überziehen von Arzneiformen und Lebensmitteln bzw. Genußmitteln mit diesen Polymeren beschrieben sind, näher erläutert.

### BEISPIELE

### A. Herstellung der Polymeren

In den Beispielen 2 bis 10 wird die Aspartamideinheit durch die allgemeine Formel 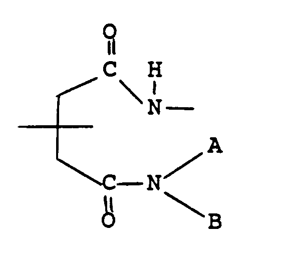
wiedergegeben.

### Beispiel 1

50 g Asparaginsäure werden in einem 1 l-Rundkolben mit 25 g 85 %iger Phosphorsäure zu einer homogenen Masse vermischt. Der Kolben wird mit einem Rotationsverdampfer verbunden und auf 100 mbar evakuiert. Man hält das Reaktionsgemisch bei laufendem Rotationsverdampfer in einem vorgeheizten Ölbad 3 Stunden bei 180 Grad C. Anschließend läßt man abkühlen, schlämmt das Reaktionsprodukt in Wasser auf und wäscht es auf einer Sinterglasnutsche mit Wasser säurefrei. Das Produkt wird im Vakuum bei 80 Grad C getrocknet. Es wird mittels IR und NMR-Spektroskopie als Polysuccinimid identifiziert.
- Ausbeute:: 35,5 g = 97,3 % d.Th.

| | | | | |
|---|---|---|---|---|
| Elementaranalyse: | ber.: | 49,5 % C | 3,1 % H | 14,4 % N |
| | gef.: | 46,5 % C | 3,7 % H | 13,5 % N |

Molekulargewicht, viskosimetrisch im DMF, nach Neri et al. im J.Med.Chem. 16, Seite 894 (1973): 90 000.

### Beispiel 2

### α,β-D,L-Poly(isopropanol)aspartamid

- A =: H
- B =: - CH₂ - CHOH - CH₃

5 g (51 mmol) Polysuccinimid werden in 30 ml DMF gelöst. Man tropft 20 ml (0,93 mol) Isopropanolamin zu und rührt 24 Stunden bei Raumtemperatur. Dann trägt man die Reaktionsmischung in das fünffache Volumen Aceton ein, saugt das ausgefallene Produkt ab und wäscht es gut mit Aceton aus. Anschließend löst man es in 20 ml Methanol und fällt erneut in einen Überschuß Aceton aus. Das Produkt wird gründlich mit Aceton gewaschen und bei 60 Grad C im Vakuum getrocknet. Ausbeute 5 g = 56 % d.Th. Die Struktur wird durch IR- und NMR-Spektroskopie bestätigt.
- Molmasse:: 14 700 (best. durch GPC, Standard Polyacrylsäure)

| | | | | |
|---|---|---|---|---|
| Elementaranalyse: | ber.: | 48,83 % C | 7.02 % H | 16,27 % N |
| | gef.: | 46,3 % C | 7,5 % H | 15,2 % N |

### Beispiel 3

### α,β-D,L-Poly(diisopropanol)aspartamid

- A und B =: - CH₂ - CHOH - CH₃

Wie Beispiel 2, jedoch unter Verwendung von 66 g 0,68 mol) Polysuccinimid, 250 ml DMF und 90,5 g (0,68 mol) Diisopropanolamin.
- Ausbeute:: 89 g = 57 % d.Th.
- Molmasse:: 22 600 (GPC, Standard Polyacrylsäure)

| | | | | |
|---|---|---|---|---|
| Elementaranalyse: | ber.: | 52,16 % C | 7,88 % H | 12,17 % N |
| | gef.: | 49,1 % C | 6,5 % H | 12,4 % N |

### Beispiel 4

### α,β-D,L-Poly(3-ethoxypropyl)aspartamid

- A =: H
- B =: - CH₂ - CH₂ - CH₂ - OC₂H₅

Wie Beispiel 2, jedoch unter Verwendung von 20 g (0,2 mol) Polysuccinimid, 50 ml DMF und 55 g (0,625 mol) 3-Ethoxypropylamin.
- Ausbeute:: 37 g = 97 % d.Th. leicht gelb gefärbter Feststoff.
Strukturbestätigung durch IR und NMR.

| | | | | |
|---|---|---|---|---|
| Elementaranalyse: | ber.: | 53,99 % C | 8,05 % H | 13,99 % N |
| | gef.: | 51,9 % C | 8,3 % H | 13,4 % N |

### Beispiel 5

### α,β-D,L-Poly(methoxyethyl)aspartamid

- A =: H
- B =: -CH₂ - CH₂ - OCH₃

Wie Beispiel 2, jedoch unter Verwendung von 30 g (0,31 mol) Polysuccinimid, 20 ml DMF und 100 g (1,33 mol) Methoxyethylamin.
- Ausbeute:: 27 g = 51 % d.Th. eines leicht gelb gefärbten Feststoffs.
Strukturbestätigung durch IR und NMR.

| | | | | |
|---|---|---|---|---|
| Elementaranalyse: | ber.: | 48,33 % C | 7.02 % H | 16,27 % N |
| | gef.: | 46,6 % C | 7,4 % H | 14,8 % N |

### Beispiel 6

### α,β-D,L-Poly(hydroxyethyl)aspartamid-co-succinimid (1:1)

- A =: H
- B =: - CH₂ - CH₂OH

Wie Beispiel 2, jedoch unter Verwendung von 5 g (51 mmol) Polysuccinimid, 30 ml DMF und 1,6 g (25,7 mmol) Ethanolamin.
- Reaktionszeit:: 1 Stunde bei Raumtemperatur
- Ausbeute:: 6,5 g = 98,5 % d.Th. eines farblosen Feststoffes.
Im NMR-Spektrum sind Hydroxyethylaspartamid- und Succinimideinheiten erkennbar im
- Molverhältnis:: 1 : 1.

| | | | | |
|---|---|---|---|---|
| Elementaranalyse : | ber.: | 47,06 % C | 5,09 % H | 16,46 % N |
| | gef.: | 44,2 % C | 5,3 % H | 15,4 % N |

### Beispiel 7

### α,β-D,L-Poly(diisopropanol)aspartamid-co-succinimid (1:1)

- A und B =: - CH₂ - CHOH - CH₃

Wie Beispiel 2, jedoch unter Verwendung von 10 g (103 mmol) Polysuccinimid, 30 ml DMF und 6,86 g (51,5 mmol) Diisopropanolamin.
- Ausbeute:: 14,2 g = 84,2 % d.Th. eines farblosen Feststoffs.
Im NMR-Spektrum sind Diisopropanolaspartamid- und Succinimideinheiten erkennbar,
- Molverhältnis:: 1 : 1.

| | | | | |
|---|---|---|---|---|
| Elementaranalyse: | ber.: | 51,38 % C | 6,42 % H | 12,84 % N |
| | gef.: | 48,5 % C | 5,6 % H | 13,0 % N |

### Beispiel 8

### α,β-D-L-Polyasparagin

- A und B =: H

10 g (103 mmol) Polysuccinimid löst man portionsweise in 200 ml 25 % wäßriger Ammoniaklösung und rührt 1,5 Stunden bei Raumtemperatur. Danach fällt man das Produkt in 1 l Aceton aus und isoliert es durch Zentrifugieren. Man erhält 11 g (93,6 % d.Th.) einer leicht gelb gefärbten Masse, die im Vakuum bei 80 Grad C getrocknet wird. Die Struktur wird durch NMR-Spektroskopie bestätigt.
- Molmasse:: 13 400 (GPC, Standard Polyacrylsäure)

| | | | | |
|---|---|---|---|---|
| Elementaranalyse: | ber.: | 42,11 % C | 5,3 % H | 24,55 % N |
| | gef.: | 38,6 % C | 5,8 % H | 20,3 % N |

### Beispiel 9

### α,β-D,L-Poly(tetrahydrofurfurylmethyl)aspartamid

- A =: H
- B =:

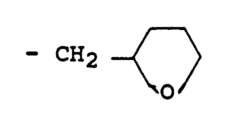

Wie Beispiel 2, jedoch unter Verwendung von 90 g (0,93 mol) Polysuccinimid, 150 ml DMF und 100 g (0,99 mol) Tetrahydrofurfurylmethylamin.
Zusätzlich wird das Produkt ein weiteres Mal in 200 ml Methanol gelöst und in einem Überschuß Aceton ausgefällt. Nach Waschen mit Aceton und Trocknen im Vakuum erhält man 26 g (13 % d.Th) Poly(tetrahydrofurfurylmethyl)aspartamid in Form eines leicht gelb gefärbten Feststoffs.
Die Struktur wird durch IR- und NMR-Spektroskopie bestätigt.

| | | | | |
|---|---|---|---|---|
| Elementaranalyse: | ber.: | 54,55 % C | 7,07 % H | 14,14 % N |
| | gef.: | 53,7 % C | 7,8 % H | 12,4 % N |

### Beispiel 10

### α,β-D,L-Poly(isononyloxypropyl)aspartamid

- A =: H
- B =: - CH₂ - CH₂ - CH₂ - OC₉H₁₉

Wie Beispiel 2, jedoch unter Verwendung von 5 g (51 mmol) Polysuccinimid, 40 ml DMF und 20 g (100 mmol) Isononyloxypropylamin.
Das Produkt wird durch Eintragen des Reaktionsgemischs in das fünffache Volumen Wasser gewonnen, mit Wasser gewaschen, erneut in 20 ml DMF aufgenommen und in einen Überschuß Wasser ausgefällt. Nach gründlichem Waschen mit dem Fällungsmittel wird das Produkt bei 60 Grad C im Vakuum bis zur Gewichtskonstanz getrocknet. Man erhält 6,2 g (40,4 % d.Th.) eines leicht gelb gefärbten Feststoffs. Die Struktur wird durch NMR-Spektroskopie bestätigt.
- Molmasse:: 38 400 (G PC, Standard: Polymethylmethacrylat)

| | | | | |
|---|---|---|---|---|
| Elementaranalyse. | ber.: | 64,43 % C | 10,1 % H | 9,4 % N |
| | gef.: | 62,1 % C | 10,1 % H | 9,4 % N |

### B. Anwendung der Polymeren als Überzugsmittel

### Beispiel 1

### Schnell zerfallender Überzug auf Tabletten

120 g Tabletten mit einem Durchmesser von 10 mm, einer Gesamthöhe von 4,3 mm und einem Gewicht von 350 mg/Stück wurden unter Rotation in einem kleinen Dragierkessel von 14 cm Durchmesser mit einer Lösung von 2,7 g Poly(isopropanol)aspartamid, nach Beispiel A2, in 23 g Methanol beschichtet. Dazu wurden kleine Portionen von etwa 0,1 - 0,2 ml auf die rotierenden Tablettenkerne geträufelt und nach kurzer Verteilung durch Einblasen eines Warmluftstromes von etwa 50 Grad C getrocknet. Es entstand ein glänzender, staubfreier Überzug, der sich leicht klebrig anfühlt, doch haften die Tabletten bei Aufbewahrung in einem geschlossenen Glas nicht aneinander. Die Tabletten zerfallen in einem Zerfallstester nach DAB in Wasser, in künstlichem Magensaft (0,1 N HCl), wie auch in künstlichem Darmsaft von pH 6,8 innerhalb von weniger als 3 Minuten und geben innerhalb von 30 Minuten das als Wirkstoff enthaltende Chinidinsulfat vollständig frei.

### Beispiel 2

### Farbiger Filmüberzug auf Tabletten

2,0 g Poly(diisopropanol)aspartamid, hergestellt nach Beispiel A3, wurden in 8 - 10 g Wasser gelöst und mit einer wäßrigen Pigmentsuspension vermischt, die 12 % Talkum, 4 % Titandioxid, 4 % Gelborange-Lack E 110, 3,6 % Polyethylenglykol 6000, 2,4 % Polysorbat (^{R} Tween 80) und 16 % Laktose enthielt. Die Mischung wurde ähnlich wie im Beispiel 1 in kleinen Portionen auf 120 g im Dragierkessel rotierenden Tabletten der gleichen Abmessungen gegeben und durch Einblasen von Warmluft mit einem Föhn getrocknet. Die farbig glänzenden, überzogenen Tabletten sind staubfrei und freifließend, sie fühlen sich glatt und nicht klebrig an und zerfallen in Wasser, künstlichem Magensaft sowie künstlichem Darmsaft innerhalb von max. 3 min. Der enthaltene Wirkstoff Chinidinsulfat wird innerhalb von 30 min vollständig freigegeben.

### Beispiel 3

5 g Poly(isopropanol)aspartamid wurden in 95 g Methanol gelöst und auf 500 g Tabletten, die in einem Dragierkessel von 25 cm Durchmesser rotieren, aufgesprüht. Die Tabletten hatten einen Durchmesser von 10 mm und eine Gesamthöhe von 3 mm, das Gewicht betrug 300 mg/Stück. Zum Aufsprühen wurde eine Luftdrucksprühpistole Walther WA I mit einem Düsendurchmesser von 1,0 mm verwendet. Der Abstand zu den im Dragierkessel rotierenden Tabletten betrug 8 - 10 cm. Getrocknet wurde mit einem Warmluftgebläse von 0,45 m³/min (cbm/min) Leistung bei einer Zulufttemperatur von 50 Grad C. Bei einer Umdrehungszahl des Dragierkessels von 30 U/min und einer kesselneigung von 30 Grad konnte bei einem Sprühdruck von 0,05 bar und einer Produkttemperatur von 33 Grad C kontinnierlich gesprüht werden. Die Sprühzeit betrug insgesamt 28 min. Die überzogenen Tabletten wurden noch 2 Stunden bei 40 Grad C nachgetrocknet. Es entstand ein glatter, gleichmäßiger, matt glänzender Überzug. Der Filmüberzug zeigte einen deutlichen geschmacksabdeckenden Effekt. Beim Lutschen der Tabletten war über mind. 10 sec. kein bitterer Geschmack wahrnehmbar. Die Tabletten zerfielen in Wasser und künstlichem Magensaft innerhalb von max. 1 Minute. Die Härte und die Abriebfestigkeit der überzogenen Tabletten war gegenüber dem unüberzogenen Ausgangsmaterial deutlich höher.

### Beispiel 4

### Filmüberzug auf Pellets

5 kg Pellets von 0,5 - 1,22 mm Durchmesser und einem Gehalt von 8 % Chlorphenaminmaleat wurden in einem Wirbelschichtgerät (Glatt WSG 5) von unten mit Warmluft durchströmt und auf eine Ausgangstemperatur von 33 Grad C vorgewärmt. Nun wurde mit einer Luftdruckspritzpistole, die im oberen Teil des Wirbelbettes fixiert war, eine Lösung von 125 g Poly(diisopropanol)aspartamid in 1 152 g Wasser aufgesprüht. Dabei wurde die Temperatur der Zuluft bei etwa 40 - 45 Grad C gehalten und bei beginnender Agglomerationsneigung der Kerne das Sprühen gelegentlich kurz unterbrochen. Nach 46 min war die Gesamtmenge aufgesprüht und das Material über Nacht bei 40 Grad C nachgetrocknet. Die Pellets waren frei fließend und staubfrei.

### Beispiel 5

2 kg Pellets mit einem Durchmesser von 0,8 - 1,5 mm wurden in einem Wirbelschichtgerät (Glatt GPC G 1) mit Siebfiltereinsatz im Warmluftstrom von 43 - 45 Grad C gewirbelt. 50 g Poly(isononyloxypropyl)aspartamid wurden in 450 g Wasser gelöst und mit 500 g einer Pigmentsuspension aus 85 g Talkum, 25 % Titandioxid, 25 g Chinolingelb-Lack, 15 g Polyethylenglykol 6 000 und 205 g Wasser vermischt und innerhalb von 70 min unter ständigem Wirbeln im Warmluftstrom eingesprüht. Der Düsendurchmesser betrug 1,2 mm und die Sprühsuspension wurde aus einem Vorratsgefäß mittels einer Schlauchpumpe der Düse zugeführt. Nach dem Trocknen, 16 Stunden bei 40 Grad C im Umlufttrockenschrank, zeigten die Pellets einen farbig mattglänzenden Überzug. Das enthaltene Chlorphenaminmaleat wurde in einem Freigabetest - Disolution Test nach USP XXI, Apparatur 2 (Paddle-Methode) - innerhalb von 1 - 2 Stunden verzögert freigegeben.

### Beispiel 6

2 kg Theophyllin-Pulver werden in einem 5-Liter VA-Gefäß unter Rühren mit einer Lösung von 10 g Poly(ethoxypropyl)aspartamid (Herstellung siehe Beispiel A4) in einem Gemisch aus 40 g Äthanol und 60 g Wasser unter Rühren befeuchtet und durch ein Sieb mit 2 mm lichter Maschenweite gedrückt. Nach dem Trocknen erhält man ein Granulat, das mit 1 % Magnesiumstearat vermischt auf einer Tablettenpresse zu Formlingen von 10 mm Durchmesser und 3,5 mm Höhe verpreßt wurde. Dazu war ein Preßdruck von 15 kN erforderlich und die Tabletten zeigten eine Bruchfestigkeit von 120 N. Die Tabletten wurden in einem Freigabetest dann nach USP (Paddle-Gerät; s. Beispiel 5) untersucht und gaben den enthaltenen Wirkstoff verzögert innerhalb von 2 - 3 Stunden frei.

### Beispiel 7

3 kg kugelige Kaugummiformlinge mit einem Durchmesser von 15 mm wurden in einem Dragierkessel von etwa 25 cm Durchmesser bei einer Umdrehung von 15 U/min bewegt und mit einer Lösung von 30 g Poly(tetrahydrofurfurylmethyl)aspartamid (Herstellung s. Beispiel A9), in der noch 10 g Talkum suspendiert sind, in einem Gemisch aus 240 g Aceton und 60 g Wasser besprüht. Während des Sprühprozesses der mit einer Luftdruckspritzpistole mit einem Düsendurchmesser von 1,0 mm durchgeführt wurde, wurde die eingeblasene Trocknungsluft bei einer Temperatur von etwa 30 Grad C gehalten. Nach einer Sprühzeit von insgesamt 60 min. und einer Nachtrocknung von 5 min im Luftstrom der gleichen Temperatur resultierten glänzende Formlinge, die staubfrei und griffest waren und auch in Standgefäßen bei 30 Grad C nicht verklebten.

### Beispiel 8

### Herstellung von Matrixtabletten.

600 g Theophyllin-Granulat wasserfrei in einer Korngröße von 0,3 - 0,8 mm wurden mit 228 g Calziumhydrogenphosphat (Emcompress) sowie 150 g Poly(isononyloxypropyl)aspartamid, Herstellungsbeispiel A10, vermischt, wobei das Polymere vorher auf eine Korngröße unter 0,1 mm vermahlen war. Das Theophyllin-Granulat, Calciumhydrogenphosphat und das Polymere wurden zunächst 30 min im Taumelmischer vorgemischt und anschließend durch ein Sieb mit einer lichten Maschenweite von 1 mm gegeben. Diese Vormischung wurde dann nach Zugabe von 15 g Talkum und 7 g Magnesiumstearat weitere 15 min im Taumelmischer bewegt und auf einer Excenterpresse mit einem Druck von 15 - 20 kN zu Tablettem mit 12 mm Durchmesser und einem Wölbungsradius von 25 mm verpreßt. Die Bruchfestigkeit betrug 100 N; im Freigabetest nach USP (Paddle-Methode; s. Beispiel 5) geben die Tabletten unter langsamem Zerfall den Wirkstoff innerhalb von 4 Stunden zu mehr als 80 % frei.

## Patentansprüche

1. Verwendung von Umsetzungsprodukten von Polysuccinimid, die filmbildend sind und Struktureinheiten entsprechend der Formel I 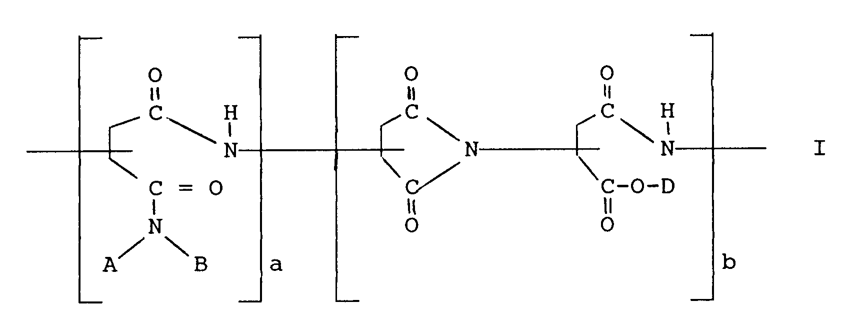 enthalten, in der
A = H oder ein Alkylrest bzw. ein Alkylenrest mit 1 bis 8 C-Atomen ist, der auch verzweigt und noch mit cycloaliphatischen oder aromatischen Resten, wobei der ringförmige Substituent auch Heteroatome enthalten kann, oder mit R-O-Gruppen, wobei R = H oder eine (gegebenenfalls verzweigte) Alkylgruppe oder Cycloalkylgruppe mit 1 bis 10 C-Atomen sein kann, substituiert sein kann,
B = H oder ein wie unter A definierter Alkyl- bzw. Alkylenrest ist, der gleich oder verschieden von A sein kann,
D = H oder NH₄ oder 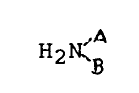 mit den oben angegebenen Bedeutungen von A und B oder ein Alkali und
a = 0,2 bis 1 und
b = 0,8 bis O
sind, als Mittel zum Einschließen von therapeutischen Wirkstoffen in Arzneiformen sowie zum Umhüllen von Lebensmitteln und Genußmitteln.

2. Verwendung von Umsetzungsprodukten nach Anspruch 1, dadurch gekennzeichnet, daß darin 30 bis 100 % und vor allem 50 bis 95 % der Succinimideinheiten zu Asparaginsäureamideinheiten umgesetzt sind.

3. Verwendung von Umsetzungsprodukten nach den Ansprüchen 1 und 2, dadurch gekennzeichnet, daß sie durch Umsetzung von Polysuccinimid mit Isopropanolamin und/oder Diisopropanolamin erhalten werden.

4. Verwendung von Umsetzungsprodukten nach den Ansprüchen 1 und 2, dadurch gekennzeichnet, daß sie durch Umsetzung von Polysuccinimid mit 3-Ethoxypropylamin erhalten werden.

5. Verwendung von Umsetzungsprodukten nach den Ansprüchen 1 und 2, dadurch gekennzeichnet, daß sie durch Umsetzung von Polysuccinimid mit Isononyloxypropylamin erhalten werden.

6. Verwendung von Umsetzungsprodukten nach den Ansprüchen 1 bis 5 zum Überziehen von Arzneiformen, wie Tabletten oder Pellets, oder Genuß- bzw. Lebensmittelzubereitungen mit Filmen.

7. Verwendung von Umsetzungsprodukten nach den Ansprüchen 1 bis 5 zur Herstellung von Umhüllungs- bzw. Verpackungsformen für Arzneimittel oder Lebensmittel bzw. Genußmittel.

8. Verwendung von Umsetzungsprodukten nach den Ansprüchen 1 bis 5 zum Überziehen von Kapseln.

9. Verwendung von Umsetzungsprodukten nach den Ansprüchen 1 bis 5 für die Herstellung von Arzneikapseln.

10. Verwendung von Umsetzungsprodukten nach den Ansprüchen 1 bis 5 für die Herstellung von transdermal therapeutisch wirkenden Systemen.

11. Verwendung von Umsetzungsprodukten nach den Ansprüchen 1 bis 5 als Matrixbildner.

12. Verwendung von Umsetzungsprodukten nach den Ansprüchen 1 bis 5 für die Herstellung von Folien.

## Claims

1. Use of reaction products of polysuccinimide, which form films and contain structural units according to Formula I 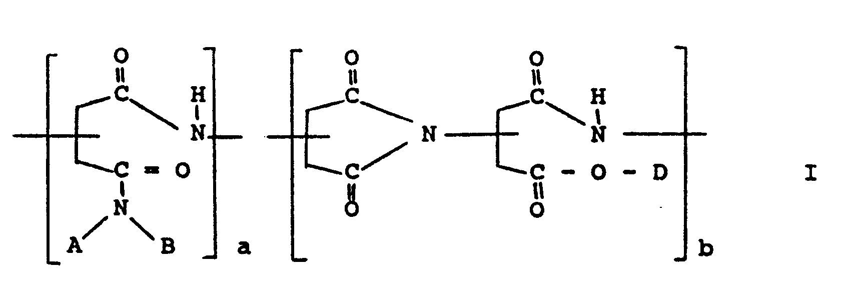 wherein
A = H or an alkyl group, or an alkylene group having 1 to 8 C-atoms, which may also be branched and may be further substituted by cycloaliphatic or aromatic groups wherein the ring-shaped substituent may also contain heteroatoms, or by R-O groups wherein R = H or an (optionally branched) alkyl group or cycloalkyl group having 1 to 10 C-atoms,
B = H or an alkyl or alkylene group as defined under A, which may be identical to or different from A,
D = H or NH₄ or 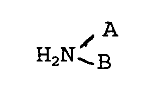 with the meanings of A and B as given above, or an alkali and
a = 0.2 to 1 and
b = 0.8 to 0,
as agents for the inclusion of therapeutically active substances in pharmaceutical preparations as well as for coating foodstuffs and stimulants.

2. Use of reaction products according to claim 1, characterised in that 30 to 100%, more particularly 50 to 95% of the succinimide units contained therein have been converted into aspartic acid amide units.

3. Use of reaction products according to claims 1 and 2, characterised in that they are obtained by reacting polysuccinimide with isopropanolamine and/or diisopropanolamine.

4. Use of reaction products according to claims 1 and 2, characterised in that they are obtained by reacting polysuccinimide with 3-ethoxypropylamine.

5. Use of reaction products according to claims 1 and 2, characterised in that they are obtained by reacting polysuccinimide with isononyloxypropylamine.

6. Use of reaction products according to claims 1 to 5 for coating pharmaceutical preparations such as tablets or pellets, or stimulants and foodstuffs with films.

7. Use of reaction products according to claims 1 to 5 for producing coating or packaging preparations for pharmaceutical compositions, foodstuffs or stimulants.

8. Use of reaction products according to claims 1 to 5 for coating capsules.

9. Use of reaction products according to claims 1 to 5 for preparing pharmaceutical capsules.

10. Use of reaction products according to claims 1 to 5 for preparing transdermally active therapeutic systems.

11. Use of reaction products according to claims 1 to 5 for forming matrices.

12. Use of reaction products according to claims 1 to 5 for preparing films.

## Revendications

1. Utilisation de produits de réaction de polysuccinimide, qui sont filmogènes et contiennent des motifs structurels correspondant à la formule I 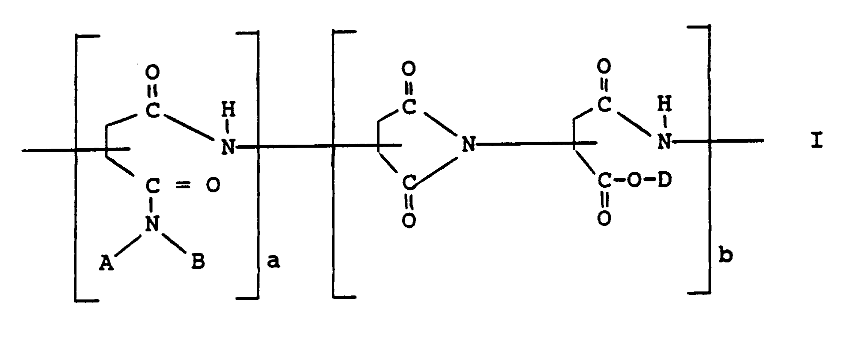 dans laquelle
A est H ou un reste alkyle ou alkylène ayant 1 à 8 atomes de carbone, qui peut être ramifié et peut encore être substitué par des restes cycloaliphatiques ou aromatiques, le substituant cyclique pouvant également contenir des hétéroatomes, ou par des groupes R-O-, R pouvant représenter H ou un groupe alkyle (éventuellement ramifié) ou cycloalkyle ayant 1 à 10 atomes de carbone,
B est H ou un reste alkyle ou alkylène tel que défini sous A, qui peut être identique à ou différent de A,
D représente H ou NH₄ ou 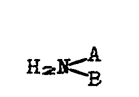 avec les significations mentionnées ci-dessus pour A et B, ou un alcali et
a est compris entre 0,2 et 1 et
b est compris entre 0,8 et 0,
comme agents pour renfermer des matières actives thérapeutiques dans des formes médicamenteuses, ainsi que pour envelopper des aliments et des friandises.

2. Utilisation de produits de réaction selon la revendication 1, caractérisée en ce que 30 à 100%, et en particulier 50 à 95% des motifs succinimide sont convertis en motifs asparaginimide.

3. Utilisation de produits de réaction selon la revendication 1 ou 2, caractérisée en ce qu'ils sont obtenus par réaction de polysuccinimide avec l'isopropanolamine et/ou la diisopropanolamine.

4. Utilisation de produits de réaction selon la revendication 1 ou 2, caractérisée en ce qu'ils sont obtenus par réaction de polysuccinimide avec la 3-éthoxypropylamine.

5. Utilisation de produits de réaction selon la revendication 1 ou 2, caractérisée en ce qu'ils sont obtenus par réaction de polysuccinimide avec l'isononyloxypropylamine.

6. Utilisation de produits de réaction selon l'une quelconque des revendications 1 à 5 pour revêtir des formes médicamenteuses, telles que des comprimés ou des pellets, ou des préparations de friandises ou alimentaires, avec des films.

7. Utilisation de produits de réaction selon l'une quelconque des revendications 1 à 5 pour préparer des enrobages ou des emballages pour des médicaments, des aliments ou des friandises.

8. Utilisation de produits de réaction selon l'une quelconque des revendications 1 à 5 pour revêtir des capsules.

9. Utilisation de produits de réaction selon l'une quelconque des revendications 1 à 5 pour la préparation de capsules médicamenteuses.

10. Utilisation de produits de réaction selon l'une quelconque des revendications 1 à 5 pour la préparation de systèmes transdermiques thérapeutiquement actifs.

11. Utilisation de produits de réaction selon l'une quelconque des revendications 1 à 5 comme précurseurs de matrices.

12. Utilisation de produits de réaction selon l'une quelconque des revendications 1 à 5 pour la préparation de feuilles.
